(19) Europäisches Patentamt
European Patent Office
Office européen des brevets

(11) Veröffentlichungsnummer: **0 116 828**
**B1**

(12) # EUROPÄISCHE PATENTSCHRIFT

(45) Veröffentlichungstag der Patentschrift: **03.12.86**

(51) Int. Cl.⁴: **C 07 D 233/90**

(21) Anmeldenummer: **84100169.6**

(22) Anmeldetag: **10.01.84**

(54) **Verfahren zur Herstellung von Imidazol-4,5-dicarbonsäure.**

(30) Priorität: **20.01.83 DE 3301717**

(43) Veröffentlichungstag der Anmeldung: **29.08.84 Patentblatt 84/35**

(45) Bekanntmachung des Hinweises auf die Patenterteilung: **03.12.86 Patentblatt 86/49**

(84) Benannte Vertragsstaaten: **BE CH DE FR GB IT LI**

(56) Entgegenhaltungen:
**EP-A- 0 000 373**
**DE-A- 2 059 042**
**DE-A- 2 618 756**
**CHEMICAL ABSTRACTS, Band 90, Nr. 23, 4. Juni 1979, Seite 665, Nr. 186948b, Columbus, Ohio, USA**

(73) Patentinhaber: **BASF Aktiengesellschaft**
**Carl-Bosch-Strasse 38**
**D-6700 Ludwigshafen (DE)**

(72) Erfinder: **Dockner, Toni, Dr.**
**Grossgasse 6**
**D-6701 Meckenheim (DE)**
Erfinder: **Kempe, Uwe, Dr.**
**Danziger Strasse 9**
**D-6701 Dannstadt-Schauernheim (DE)**
Erfinder: **Frank, Anton**
**Bannwasserstrasse 72**
**D-6700 Ludwigshafen (DE)**

**EP 0 116 828 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Imidazol-4,5-dicarbonsäure ist ein wertvolles Zwischenprodukt für Pharmaka. So üben z. B. die aus dieser Säure leicht herstellbaren Amide eine stimulierende Wirkung auf das Zentralnervensystem aus und haben auch als Sedativum therapeutische Anwendung gefunden. Imidazol-4,5-dicarbonsäure wird auch für die Herstellung von 4-Amino-imidazol-5-carbonsäure verwendet, die ein Zwischenprodukt für Purinsynthesen darstellt. Imidazol-4,5-dicarbonsäure ist auch ein wichtiger Baustein für halbsynthetische Penicilline und Cephalosporine mit ausgezeichneter Wirksamkeit.

Imidazol-4,5-dicarbonsäure läßt sich z. B. durch Umsetzung von Weinsäuredinitrat mit Ammoniak und Formaldehyd in wäßriger Lösung herstellen (Ann. Chem. Phys. 24 (1891), S. 523 bis 525). Man kann diese Säure auch durch Oxidation von Benzimidazol mit Kaliumbichromat oder Kaliumpermanganat gewinnen (Z. obsc. Chem. 26 (1956), S. 455).

Da diese Verfahren den Nachteil haben, daß sie von sehr teuren Ausgangsmaterialien ausgehen, bestand die Aufgabe, eine Synthese zu finden, die es gestattet, dieses gesuchte Zwischenprodukt auf wirtschaftlicherem Wege herzustellen.

Aus der DE-A-2059042 ist es bekannt, längerkettige vicinale Diole mit Salpetersäure bei einer Temperatur von 40 °C bis ca 100 °C unter Aufspaltung zu oxidieren und in DE-A-2618756 ist die Umsetzung von arylsubstituierten Imidazolen mit Formaldehyd in basischem Medium beschrieben.

Es wurde nun gefunden, daß man Imidazol-4,5-dicarbonsäure besonders vorteilhaft dadurch herstellen kann, daß man Imidazol bei höherer Temperatur mit der 2- bis 5-fachen molaren Menge an Formaldehyd umsetzt und das Reaktionsgemisch bei 100 bis 140 °C mit Salpetersäure behandelt.

Dieses neue Verfahren ist wegen seiner wohlfeilen Ausgangsstoffe besonders wirtschaftlich. Es ermöglicht die Gewinnung von Imidazol-4,5-dicarbonsäure auf einfache Weise und in guten Ausbeuten.

Das erfindungsgemäße Verfahren läßt sicht mit folgendem allgemeinen Schema beschreiben :

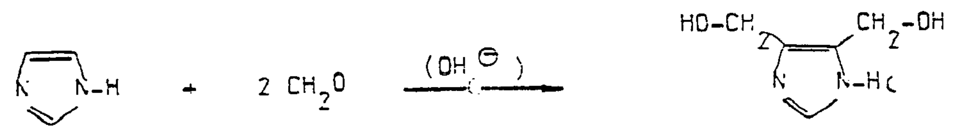


(I) (II)

II

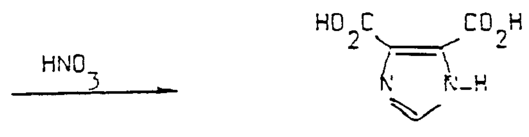


(III)

Die Verbindung (II) steht für ein Gemisch aus Imidazolderivaten unterschiedlichen Hydroximethylierungsgrades. Das Reaktionsgemisch enthält nach HPLC-Analyse 4,5-Bishydroximethylimidazol und 1,2,4,5-Tetrahydroximethylimidazol neben wenig 1-Hydroximethyl- und 4-Hydroximethylimidazol. Darüber hinaus enthält es oligomere Kondensationsprodukte, die, wie von Hydroximethylverbindungen bekannt, unter den Reaktionsbedingungen Sekundärreaktionen erleiden.

Nach dem neuen Verfahren wird Imidazol bei höherer Temperatur, vorzugsweise bei 80 bis 120 °C mit der 2- bis 5-fachen, vorzugsweise 2,5- bis 3,5-fachen molaren Menge an Formaldehyd umgesetzt. Man kann anstelle von Formaldehyd auch Paraformaldehyd verwenden. Zweckmäßigerweise arbeitet man in wäßriger Lösung, wobei man den Formaldehyd in der handelsüblichen 30 bis 40 %igen wäßrigen Lösung anwendet. Vorteilhaft führt man diese Umsetzung in Gegenwart von starken Basen, wie den Hydroxiden der Alkali- oder Erdalkalimetalle, bevorzugt mit KOH oder NaOH durch. Die Basen werden im allgemeinen in Mengen von 30 bis 100, vorzugsweise 45 bis 55 Mol.%, bezogen auf das Imidazol, eingesetzt. Die Reaktionszeit beträgt etwa 1 bis 3 Stunden. Die Umsetzung während der ersten Stufe läßt sich z. B. durch HPLC-Messung kontrollieren.

Das durch diese Umsetzung erhaltene Reaktionsgemisch wird nun unmittelbar oder nach Aufkonzentrierung bei 100 bis 140 °C, vorzugsweise 130 bis 135 °C mit Salpetersäure behandelt. Auch diese Verfahrensstufe führt man vorzugsweise in waßriger Lösung durch. Das Molverhältnis von Salpetersäure zu Imidazol beträgt zweckmäßigerweise 15 bis 20 : 1. Die Reaktionszeit beträgt etwa 6 bis 10 Stunden. Man verfährt z. B. so, daß man 65 %ige Salpetersäure in einem Rührkolben vorlegt, zum Sieden erhitzt, und in die siedende Säure langsam die wäßrige Lösung der Oligohydroximethylimidazole gibt. Während der Reaktion wird Wasser abdestilliert, und das wäßrige Reaktionsgemisch aufkonzentriert. Nach Ende der Reaktion wird das Gemisch abgekühlt, dabei kristallisiert reine Imidazol-4,5-dicarbonsäure aus. Die

Säure wird abfiltriert, mit Wasser gewaschen und getrocknet. Aus dem Filtrat kann man durch Einstellung des pH-Wertes auf 4 eine zweite Fraktion an Imidazol-4,5-carbonsäure erhalten. Man kann das Filtrat nach Ergänzung mit frischer Salpetersäure erneut für das Verfahren einsetzen.

Beispiel 1

68 g Imidazol werden in 245 g einer 37 gew.%igen wäßrigen Formaldehydlösung gelöst. Die Lösung wird mit 28 g Kaliumhydroxid versetzt und 3 Stunden am Rückfluß erhitzt. In einem Rührkolben, der mit einer 50 cm-Kolonne mit Rücklaufteiler, Kontaktthermometer und Tropftrichter ausgerüstet ist, werden 1,3 l 65 %ige Salpetersäure zum Sieden erhitzt. Das Reaktionsgemisch, das die Oligo-Hydroxymethylimidazol-Verbindungen enthält, wird nun innerhalb 1 Stunde in die siedende Salpetersäure eingetropft. Unter starker Entwicklung nitroser Gase kocht das Reaktionsgemisch am Rückfluß. 30 Minuten nach Beendigung des Zutropfens hört die Entwicklung der nitrosen Gase auf. Man destilliert bei 100 bis 102 °C und einem Rücklaufverhältnis von 10 : 1 innerhalb von 5 bis 6 Stunden etwa 500 g einer 5 bis 8 %igen Salpetersäure ab. Das Reaktionsgemisch wird im Eisbad gekühlt. Die ausgeschiedenen Kristalle werden abgesaugt, mit 150 ml Wasser gewaschen und getrocknet. Man erhält 50 g Imidazol-4,5-dicarbonsäure mit einer Reinheit von 96,6 % (nach HPLC) und einem Schmelzpunkt von 287 bis 289 °C (Zers.). Das Filtrat wird unter Kühlung mit Ammoniakwasser neutralisiert und mit Ameisensäure auf pH 4 gestellt. Dabei kristallisieren weitere 76 g Imidazol-4,5-dicarbonsäure mit einer Reinheit von 95,2 % aus. Die Gesamtausbeute beträgt 120 g Imidazol-4,5-dicarbonsäure entsprechend 77,0 % der Theorie.

Beispiel 2

Man verfährt wie in Beispiel 1 beschrieben, wobei man jedoch nach Abtrennung der 68 g Imidazol-4,5-dicarbonsäure das salpetersaure Filtrat nicht neutralisiert, sondern mit frischer 65 %iger Salpetersäure auf ein Volumen von 1,3 l auffüllt und in der Destillationsapparatur zum Sieden erhitzt. Nach Beendigung der Entwicklung nitroser Gase wird das gemäß Beispiel 1 hergestellte Ausgangsgemisch, das die Oligohydroximethylimidazol-Verbindungen enthält, wie in Beispiel 1 beschrieben, zugetropft. Dann wird das entstandene Reaktionswasser als ca. 5 bis 8 %ige Salpetersäure abdestilliert und die gebildete Imidazol-4,5-dicarbonsäure wie beschrieben abgetrennt. Dieser Vorgang läßt sich beliebig oft wiederholen. Das anfallende Kaliumnitrat wird durch Waschen der auskristallisierten Imidazol-4,5-dicarbonsäure ausgeschleust.

Die Ausbeuten an Imidazol-4,5-dicarbonsäure liegen bei 75 bis 80 %, bezogen auf das eingesetzte Imidazol.

**Patentansprüche**

1. Verfahren zur Herstellung von Imidazol-4,5-dicarbonsäure, dadurch gekennzeichnet, daß man Imidazol bei höherer Temperatur mit der 2- bis 5-fachen molaren Menge an Formaldehyd umsetzt und das Reaktionsgemisch bei 100 bis 140 °C mit Salpetersäure behandelt.

2. Verfahren nach Anspruch 1, dadurch gekennzeichnet, daß man die Umsetzung von Imidazol mit Formaldehyd in Gegenwart einer starken Base durchführt.

3. Verfahren nach Anspruch 1, dadurch gekennzeichnet, daß man die Umsetzung von Imidazol mit dem Formaldehyd in wäßriger Lösung durchführt.

4. Verfahren nach Anspruch 1, dadurch gekennzeichnet, daß man die Umsetzung von Imidazol mit dem Formaldehyd bei 80 bis 120 °C durchführt.

5. Verfahren nach Anspruch 2, dadurch gekennzeichnet, daß man als starke Base ein Hydroxid der Alkali- oder Erdalkalimetalle verwendet.

6. Verfahren nach Anspruch 1, dadurch gekennzeichnet, daß das Molverhältnis von Salpetersäure zu Imidazol 15 bis 20 : 1 beträgt.

**Claims**

1. A process for the preparation of imidazole-4,5-dicarboxylic acid, wherein imidazole is reacted with a 2-fold to 5-fold molar amount of formaldehyde at elevated temperatures, and the reaction mixture is treated with nitric acid at from 100 to 140 °C.

2. A process as claimed in claim 1, wherein the reaction of imidazole with formaldehyde is carried out in the presence of a strong base.

3. A process as claimed in claim 1, wherein the reaction of imidazole with formaldehyde is carried out in aqueous solution.

4. A process as claimed in claim 1, wherein the reaction of imidazole with formaldehyde is carried out at from 80 to 120 °C.

5. A process as claimed in claim 2, wherein the strong base used is an alkali metal or alkaline earth

metal hydroxide.

6. A process as claimed in claim 1, wherein the molar ratio of nitric acid to imidazole is from 15 : 1 to 20 : 1.

**Revendications**

1. Procédé de préparation de l'acide imidazole-4,5-dicarboxylique, caractérisé en ce que l'on fait réagir l'imidazole à température accrue avec une quantité deux à cinq fois molaire d'aldéhyde formique, puis l'on traite le mélange réactionnel entre 100 et 140 °C par l'acide nitrique.

2. Procédé suivant la revendication 1, caractérisé en ce que la réaction de l'imidazole et de l'aldéhyde formique est réalisée en présence d'une base forte.

3. Procédé suivant la revendication 1, caractérisé en ce que la réaction de l'imidazole et de l'aldéhyde formique est réalisée en solution aqueuse.

4. Procédé suivant la revendication 1, caractérisé en ce que la réaction de l'imidazole et de l'aldéhyde formique est réalisée entre 80 et 120 °C.

5. Procédé suivant la revendication 2, caractérisé en ce que la base forte est choisie parmi les hydroxydes de métaux alcalins et alcalino-terreux.

6. Procédé suivant la revendication 1, caractérisé en ce que le rapport molaire acide nitrique-imidazole est compris entre 15 : 1 et 20 : 1.